# FASCICULE DE BREVET EUROPEEN

(11) **EP 1 647 247 B1**
(45) Date de publication et mention de la délivrance du brevet: **11.03.2009**
(21) Numéro de dépôt: 05290611.2
(22) Date de dépôt: 21.03.2005
(51) Int. Cl.: A61F 5/02

(54) **Ceinture thérapeutique pour la traction et la décompression de la colonne vertébrale**
Therapeutischer Gürtel zur Streckung und Dekompression der Wirbelsäule
Therapeutic belt for the traction and decompression of the vertebral column

(30) Priorité: 12.10.2004 US 214993
(43) Date de publication de la demande: 19.04.2006
(73) Titulaire: Burgueno, Edward, 75006 Paris (FR)
(72) Inventeur: Burgueno, Edward, 75006 Paris (FR)

(56) Documents cités:
- US-A- 3 561 434
- US-A- 3 572 327
- US-A- 3 587 570
- US-A- 4 099 524
- US-A- 4 865 022

## Description

La présente invention concerne une ceinture conçue pour effectuer la traction et la décompression des vertèbres de la région dorso-lombaire, pour le soulagement et la prévention des lombalgies.

Les moyens actuels disponibles sur le marché sont des ceintures, élastiques ou rigides, portées pour le maintien lombaire. Ces moyens ne sont pas efficaces pour exercer des forces de traction actives sur la colonne vertébrale.

US-A-4 099 524, montre une ceinture type "sacro-lumbar" avec deux anses latérales.

La présente invention consiste en une double bande de toile de coton ou synthétique non extensible, formée de deux pièces égales assemblées (1) avec du scratch aux extrémités (2) pour l'accrochage antérieur.

La ceinture est dessinée en forme de long rectangle gentiment courbé dans toute sa longueur. Ce dessin arrondi fait qu'une fois accrochée, elle forme un cône tronqué, plus étroite au niveau du contour supérieur et plus large au niveau du contour inférieur. Ce dessin permet également à la ceinture de rester sur le pelvis, appuyée sur la crête iliaque, sans possibilité de glissement vers le bas.

Les éléments essentiels sont les deux anses latérales (3) en sangle de coton ou synthétique, fortement attachées à la ceinture au niveau des hanches. Elles sont munies de deux poignées tubulaires (4) en matière plastique ou en bois, placées à 4 cm en dessous et parallèles au bord inférieur de la ceinture, de deux sangles en coton ou synthétique (5) avec fermeture bouton-pression (6) assemblées à la ceinture au dessus des poignées, pour les plier et les attacher à la ceinture.

Les anses et les poignées sont des dispositifs nécessaires et indispensables permettant au porteur de les empoigner et d'appliquer la force des bras pour pousser vers le bas. Le résultat est la traction et l'étirement de la colonne vertébrale avec la décompression des disques et des ligaments intervertébraux.

La ceinture s'adresse à un vaste secteur de la population, aux personnes sédentaires, athlétiques, à celles qui peuvent souffrir du fait de leur mode de vie, travail ou loisir.

Légère, souple et flexible, elle peut être portée pendant toutes les activités quotidiennes, même en position allongée pour une traction optimale.

Le port de la ceinture a aussi un but préventif : elle aide le maintien lombaire, l'étirement et la souplesse de la colonne vertébrale, elle contribue à l'équilibre, la posture et la relaxation musculaire.

Les dessins annexés illustrent l'invention.

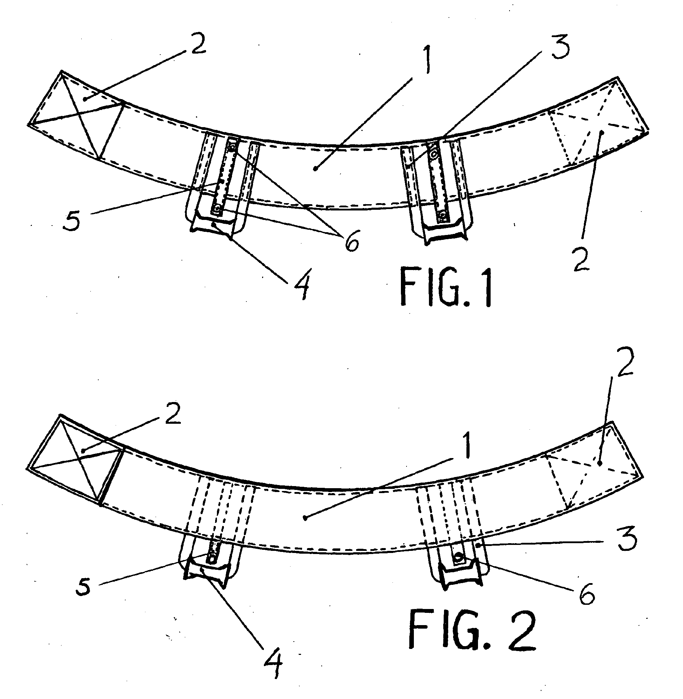

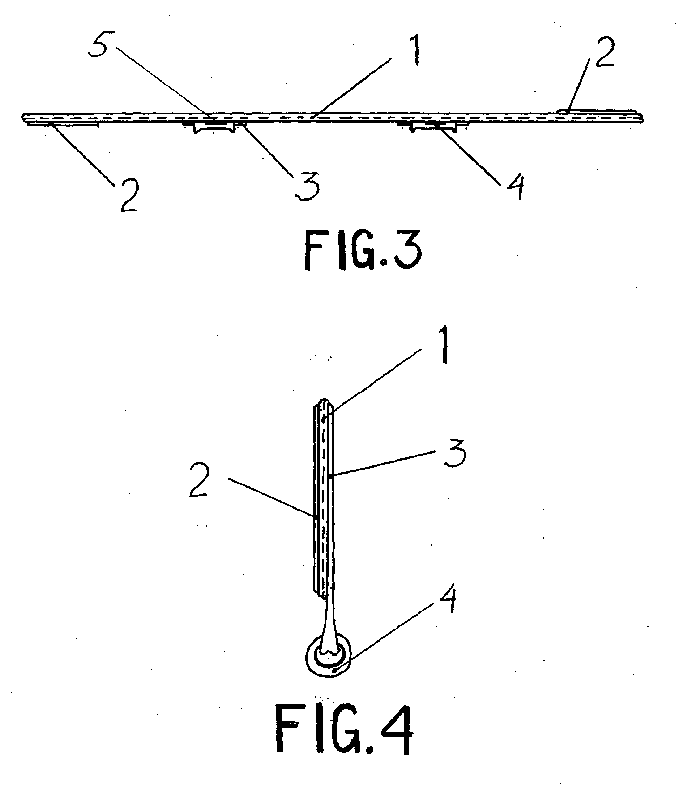

Figure 1- face externe de la ceinture; figure 2- face interne de la ceinture; figure 3- bord supérieur de la ceinture; figure 4- coupe transversale de la ceinture.

En se référant à ces deux schémas, l'invention comporte :
La ceinture (1), la fermeture scratch (2), les deux anses (3), les deux poignées (4), les sangles (5) et les boutons-pression (6).

Dimensions du prototype : 100 x 12 cm. Poignées : 12 cm de long et 2 à 2,5 cm de diamètre.

## Revendications

1. Ceinture pour effectuer la traction de la colonne vertébrale et la décompression des disques et des ligaments intervertébraux, où la ceinture est en double toile de coton ou synthétique non extensible (1) et la ceinture comporte une fermeture antérieure en scratch (2), deux anses latérales (3) en sangle de coton ou synthétique, **caractérisée en ce que** les anses sont munies de poignées tubulaires (4) en matière plastique ou en bois et **en ce que** la ceinture comporte aussi deux sangles (5) en coton ou synthétique avec des boutons-pression (6).

2. Ceinture selon la revendication 1 qui peut former dans un état fermée, un cône tronqué, qui est plus étroite au bord supérieur et plus large au bord inférieur.

## Claims

1. Belt to perform the traction of the spinal column and the decompression of the intervertebral discs and ligaments, where the belt is made of a double layer of non-elastic cotton or synthetic cloth (1), the belt comprises a frontal attachment in hook and loop (2), and two lateral loops (3) in cotton or synthetic, **characterized in that** the loops are provided with tubular handles (4) in plastic or wood and **in that** the belt also comprises two loops (5) in cotton or synthetic with snap fasteners (6).

2. Belt according to claim 1 that can form in the closed state, a truncated cone that is narrower at the upper edge and wider at the lower edge.

## Patentansprüche

1. Gurt zur Dehnung der Wirbelsäule und zur Entspannung der Bandscheiben und Bänder. Der Gurt besteht aus doppellagigem nichtelastischem Baumwollgewebe oder Synthethik (1). Der Gurt ist mit einem Klettverschluss vorne (2) und zwei seitlichen Schlaufen (3) aus Baumwollgewebe oder Synthetik versehen, ausgerüstet mit zwei speziellen Griffen aus Plastik oder Holz (4). Zusätzlich zwei Aufhänger mit Druckknöpfen (5) aus Baumwollgewebe oder Synthethik (6).

2. Gurt, analog Produktbeschreibung 1, der im geschlossenen Zustand eine konische Form bildet: obere Kante enger, untere Kante weiter.
